# EUROPEAN PATENT APPLICATION

(11) **EP 1 811 773 A2**
(43) Date of publication of application: **25.07.2007**
(21) Application number: 07100336.2
(22) Date of filing: 10.01.2007
(51) Int. Cl.: H04N 5/445

(54) **Method and device for providing brief information on data broadcasting service in digital multimedia broadcasting receiving terminal**

(30) Priority: 24.01.2006 KR 20060007505
(71) Applicant: Samsung Electronics Co., Ltd., Suwon-si 442-742 Gyeonggi-Do (KR)
(72) Inventor: Kim, Sun-Mi, c/o Samsung Electronics Co. Ltd.,, Suwon-si, Gyeonggi-do (KR); Kim, Young-Jip, c/o Sansung Electronics, Co. Ltd., Suwon-si, Gyeonggi-do (KR)
(74) Representative: Zimmer, Franz-Josef

(57) **Abstract**

Disclosed is a method for providing brief information on a data broadcasting service in a Digital Multimedia Broadcasting (DMB) receiving terminal, which includes extracting brief information on a data broadcasting service from received DMB signal to sort and store the brief information for each channel and program, and displaying brief information on a relative data broadcasting service together with information for each program when requesting Electronic Program Guide (EPG) information. Accordingly, the convenience of use of such a terminal may be increased.

## Description

The present invention relates generally to a digital multimedia broadcasting service, and more particularly to a method and a device for providing brief information on a data broadcasting service in a digital multimedia broadcasting receiving terminal.

A Digital Multimedia Broadcasting (hereinafter DMB) service provides both a main video/audio service as well as a data broadcasting service. Additional information related to programs provided by the main service or useful daily life information for users is also provided.

The data broadcasting service includes a data service that is linked with each DMB channel or independently serviced, and a download application service that can be executed using a downloaded arbitrary application module. Although the data broadcasting service is independently provided, it is mainly for linking with DMB channels. As an example of the data broadcasting service, a video service, which is a main service, and a Broadcasting Web Site (BWS) service may be provided within one ensemble. A DMB system links two services to transmit information, and if a user of a DMB receiving terminal selects a BWS service linked with a current service while watching a video service through the DMB receiving terminal, the user can receive a main video service together with a data broadcasting service. For example, when the main video service is a movie, text information on topics related to the movie such as the director, an actor or the background, may be provided simultaneously with the main service through the data broadcasting, and the web site of an Internet shopping mall where properties used in the movie are sold may be provided in a BWS format to link with a main channel.

If a DMB system supplies a plurality of application modules in a case of the download application service, the DMB receiving terminal automatically downloads an arbitrary application in the user's selection or relative channel connection. If the download is completed, data broadcasting is provided in accordance with the user's selection or by executing the automatically downloaded application. Games or reproduction programs capable of reproducing multimedia data with a specific format may be examples of the aforementioned application modules.

However, the presence of the data broadcasting service provided to link with DMB channels as described above can be known only when a user is connected to a correspondent DMB channel through a DMB receiving terminal. In other words, without connecting to the correspondent channel, the DMB receiving terminal can neither know what data broadcasting is being serviced while linking with the correspondent channel, nor whether the data broadcasting is being serviced. Moreover, even when the DMB receiving terminal is connected to the correspondent DMB channel, there is no user interface through which a user can intuitively obtain information on applications provided through the download application service.

Meanwhile, the DMB system provides an Electronic Program Guide (EPG) service in which EPG information containing current program information and program information according each ensemble or time is sent to a DMB receiving terminal. The DMB receiving terminal provides all the program information for each channel to a user through the EPG information received from the DMB system. However, the EPG information does not provide information on a data service provided while linking with an arbitrary DMB channel. The EPG information only provides information on a data service independently provided without linking with a DMB channel.

Recently, data broadcasting has been actively researched in the DMB service in connection with a mobile concept, because data broadcasting can increase user convenience and provide a vast array of contents beyond video/audio broadcasting. Accordingly, data broadcasting will provide an increasingly important aspect of DMB service in future, and DMB EPG service will also play an important role as an interface through which a vast amount of channel information in DMB is viewed when selected by a user.

Accordingly, the present invention has been made to solve the above-mentioned problems occurring in the prior art. It is the object of the present invention to provide a method and a device for providing brief information on a data broadcasting service, which can effectively and conveniently provide a user with information on a data broadcasting service provided to link with a DMB channel.

This object is solved by the subject matter of the independent claims.

Preferred embodiments are defined in the dependent claims.

It is an aspect of the present invention to provide a method and a device for providing brief information on a data broadcasting service, which can provide brief information on a data broadcasting service linked with EPG information for each channel when providing the EPG information.

It is a further aspect of the present invention to provide a method for providing brief information on a data broadcasting service, which can execute a corresponding data broadcasting service when selecting brief information on a data broadcasting service provided with EPG information.

In order to accomplish the above, according to the present invention, there is provided a method for providing brief information on a data broadcasting service in a DMB receiving terminal, including extracting the brief information on a data broadcasting service from a received DMB signal so as to sort and store the information for each channel and program, and displaying the brief information on a relative data broadcasting service together with information for each program when requesting EPG information.

According to the present invention, there is provided a device for providing brief information on a data broadcasting service in a DMB receiving terminal, including a memory for storing EPG information and brief information on a data broadcasting service, and a DMB processor for extracting the brief information on the data broadcasting service from a received DMB signal so as to sort and store the information for each channel and program, and displaying brief information on a relative data broadcasting service together with information for each program when requesting the EPG information.

The present invention will be more apparent from the following detailed description taken in conjunction with the accompanying drawings, in which:
FIG. 1 is a view illustrating a DMB system and a DMB receiving terminal according to which the present invention is applied;
FIG. 2 is a view illustrating a configuration of sub-channel FIG O/2 in a DMB service to which the present invention is applied;
FIG. 3 is a block diagram illustrating a configuration of a DMB receiving terminal to which the present invention is applied;
FIGS. 4A and 4B are flowcharts illustrating an operational process of a DMB receiving terminal according to the present invention;
FIG. 5 is a view illustrating an EPG information display screen according to the present invention;
FIGS. 6A and 6B are views illustrating a configuration of an EPG sub-window when providing a digital multimedia broadcast according to the present invention; and
FIG. 7 is a view illustrating a BWS screen linked with an arbitrary channel according to the present invention.

Hereinafter, the preferred embodiment of the present invention will be described in detail with reference to the accompanying drawings.

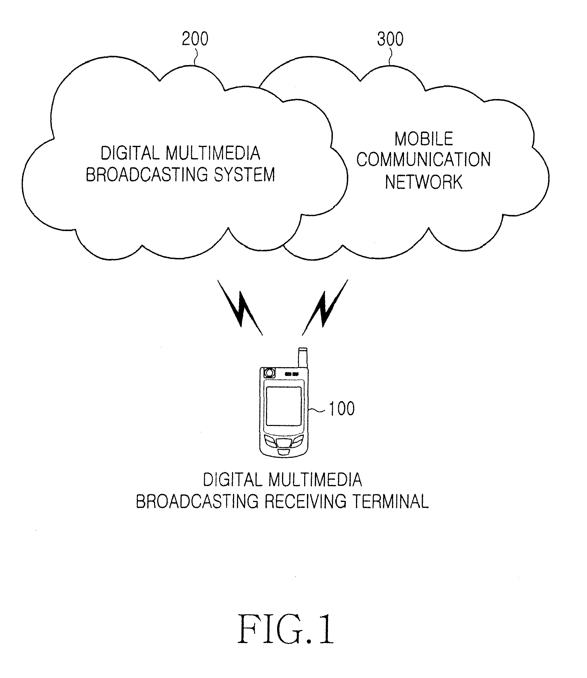

FIG. 1 is a view illustrating the DMB system and the DMB receiving terminal to which the present invention is applied. Referring to FIG. 1, the DMB system 200 provides a DMB service to a DMB receiving terminal 100. A mobile communication network 300 is linked with the DMB system 200 and provides a mobile communication service.

The DMB system 200 converts contents provided from a DMB content provider into a format possible for DMB to broadcast to DMB receiving terminals 100. Further, the DMB system 200 broadcasts Electronic Program Guide (EPG) information composed of information on a DMB program to be transmitted to the DMB receiving terminals 100. According to the present invention, the DMB system 200 transmits brief information on a data broadcasting service, which is contained in a Fast Information Channel (FIC) of DMB signals. The brief information on a data broadcasting service is brief information on contents provided through data broadcasting and includes data and application service information.

When the DMB system 200 provides the data service through an independent channel while linking with a main video/audio service, it provides the data service information through a Fast Information Group (FIG) O/2 in the FIC. In other words, the DMB system 200 provides link information related to the data service through the FIG O/2.

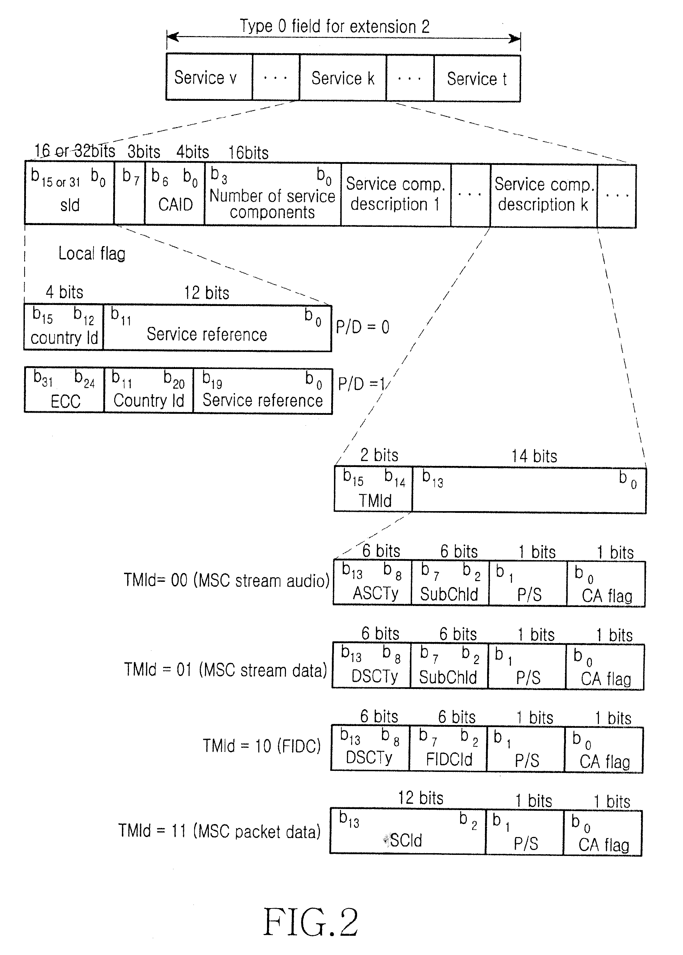

FIG. 2 is a view illustrating a configuration of sub-channel FIG O/2 in a DMB service to which the present invention is applied. Referring to FIG. 2, linked services are transmitted to one or more service components per service. Information on each of the service components is stored in each service component description field, and link information is stored in a P/S field. When the value of the P/S field is 1, it indicates that a correspondent service component is a main service (generally, a video/audio service) and when the value is zero, it indicates that a corresponding service is not a main service. Thus, link information between services can be known through the aforementioned value of the P/S field.

The application service information is brief information related to a download application service in which an arbitrary application module is provided, and includes such information as the number of applications to be downloaded, a name, download time point information, automatic/selective download sorting information and automatic/selective execution sorting information. The download time point information regards a time when a corresponding application can be downloaded or an available period when the downloaded application can be used, the automatic/selective download sorting information indicates whether the application is downloaded automatically or in accordance with user's selection when the corresponding application is connected to a relative DMB channel, and the automatic/selective execution sorting information indicates whether the application is executed automatically or in accordance with user's selection after completing the download of the correspondent application.

The DMB terminal 100 receives a DMB signal broadcasted from the DMB system 200 and is connected to the mobile communication network 300 to perform mobile communications. In addition, the DMB terminal 100 receives the EPG information. Further, according to the present invention, the DMB terminal 100 extracts brief information on a data broadcasting service from the FIC of the received DMB signal and stores it in an EPG database. When EPG data is provided on the user's demand, the DMB terminal 100 provides brief information on a data broadcasting service and download status information on a downloaded application together with the EPG data.

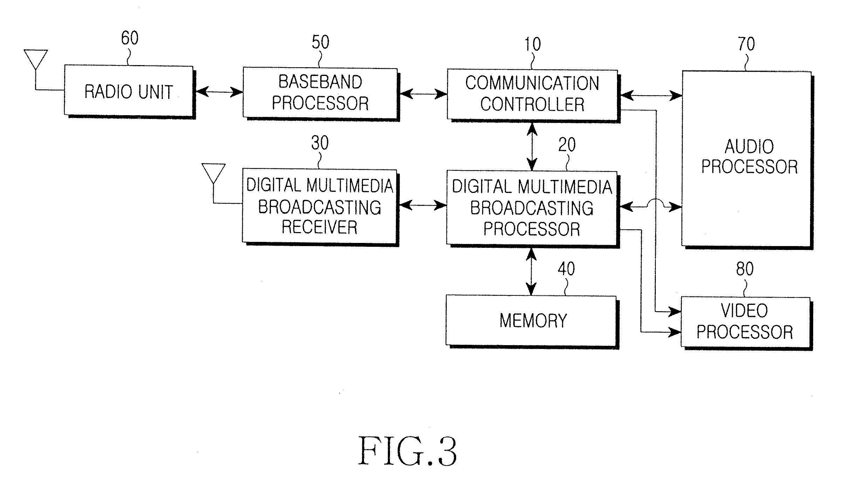

FIG. 3 is a block diagram illustrating a configuration of a DMB receiving terminal according to the present invention. As shown in FIG. 3, the DMB receiving terminal 100 includes a communication controller 10, a DMB processor 20, a DMB receiver 30, a memory 40, a baseband processor 50, a radio unit 60, an audio processor 70 and a video processor 80.

The DMB receiver 30 receives and transmits a DMB signal to the DMB processor 20 under a control of the DMB processor 20.

The DMB processor 20 decodes a DMB signal input from the DMB receiver 30 depending on key input data input from the communication controller 10. The DMB processor 20 outputs video data contained in the decoded DMB signal and DMB sound source data to the video and audio processors 80 and 70, respectively.

The communication controller 10 controls the operation of the DMB receiving terminal 100 in accordance with mobile communication performance.

The radio unit 60 transmits/receives a radio signal to/from a mobile communication station through an antenna. The radio unit 60 modulates a transmission signal input from the communication controller 10 through the baseband processor 50 into a radio signal so as to transmit it through the antenna, and demodulates a radio signal input through the antenna to provide it to the communication controller 10 through the baseband processor 50.

The baseband processor 50 processes a baseband signal transmitted/received between the radio unit 60 and the communication controller 10.

The audio processor 70 is connected to a plurality of sound source output means and a microphone so as to output sound source data input from the microphone to the communication controller 10, while outputting sound source data input from the communication controller 10 and the DMB processor 20 to the plurality of sound source output means. The plurality of sound source output means are for outputting sounds, in which a speaker, a receiver and the like are included.

The video processor 80 displays various types of images including video data input from the communication controller 10 and the DMB processor 20 under a control thereof.

The memory 40 stores programs for processing and controlling the DMB processor 20, reference data, a variety of renewable data for storage and the like, and is provided as a working memory of the DMB processor 20. The memory 40 is also provided with an EPG database to store EPG information and brief information on a data broadcasting service detected from the FIC of a DMB signal. Furthermore, , the memory 40 stores user interface program data for simultaneously providing the EPG information and the brief information on a data broadcasting service.

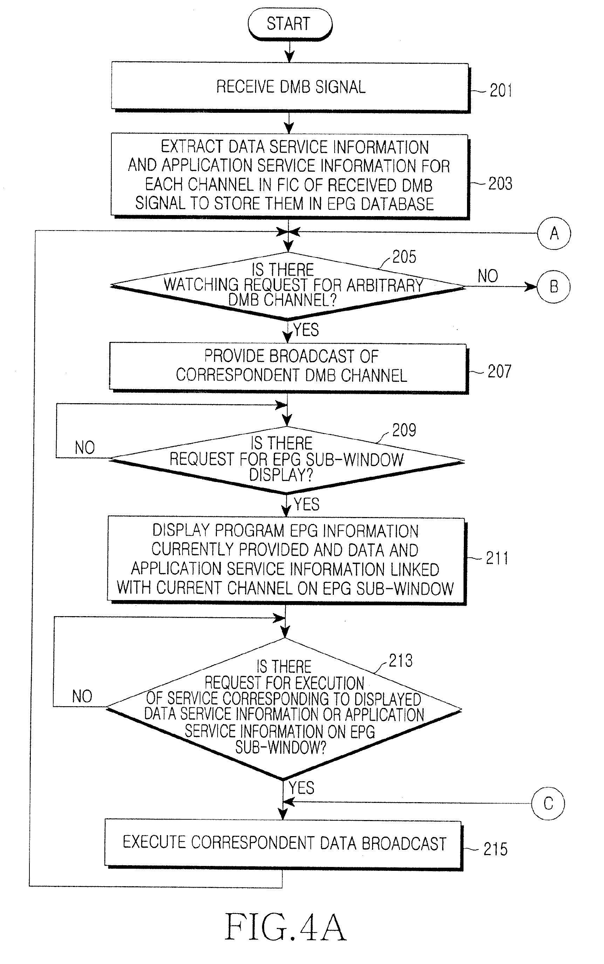

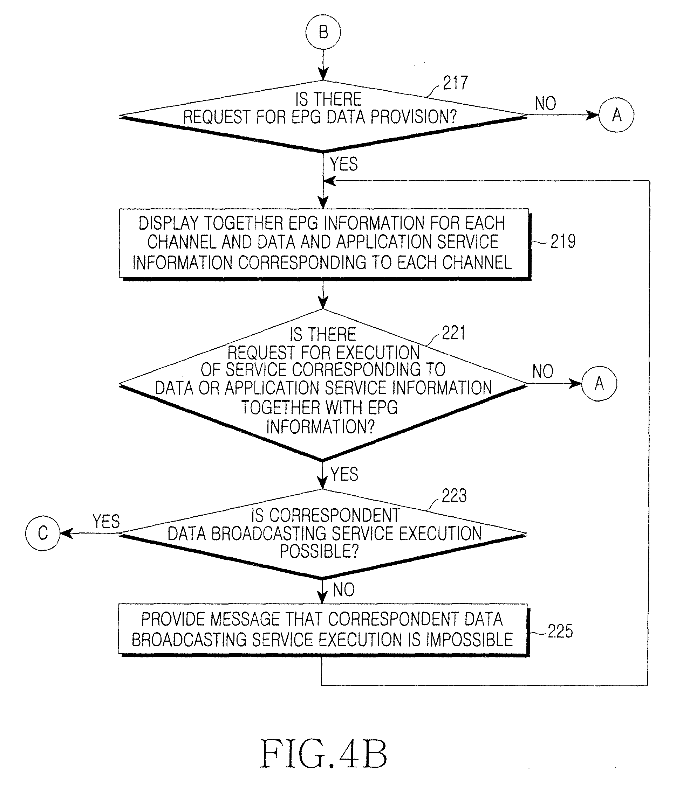

FIGS. 4A and 4B are flowcharts illustrating an operational process of a DMB receiving terminal, according to the present invention. As shown in FIGs. 3 and 4A, when the DMB processor 20 receives a DMB signal from the DMB system 200 at step 201, it proceeds to step 203. The DMB processor 20 extracts data and application service information for each channel in the FIC of the DMB signal so as to store it in an EPG database at step 203, and then proceeds to step 205. In this manner, the DMB processor 20 obtains the data service information with reference to the P/S field of an FIG O/2 in the FIC so as to store the data service information linked for each channel, and obtains the application service information contained in the FIC, i.e., the number of applications to be downloaded, a name, download time point information, automatic/selective download sorting information and automatic/selective execution sorting information so as to store them for each channel. The data and application service information are stored for each program.

Thereafter, the DMB processor 20 receives a watching request for an arbitrary DMB channel at step 205 and then proceeds to step 207 where it provides a broadcast of a DMB channel corresponding to the user's request and then proceeds to step 209. If the DMB processor 20 receives an input for requesting an EPG sub-window to be displayed from the user at step 209, it proceeds to a step 211. The EPG sub-window is displayed on a region of a lower portion in a display screen while outputting DMB broadcasting. The DMB processor 20 extracts EPG information on a program currently provided and data service information and relative application information, which are linked with a current channel, from the EPG database so as to display them on the EPG sub-window at step 211. At this time, the DMB processor 20 may extract the download status of an application provided through the download application service to display it on the EPG sub-window. The application may be downloaded depending on its type or setup automatically or in accordance with user's selection when connecting with a channel. Thus, if an application download is executed according to the present invention, the DMB processor 20 stores application download status information together with relative application service information in the EPG database and provides it together with EPG data when the user requests the EPG data.

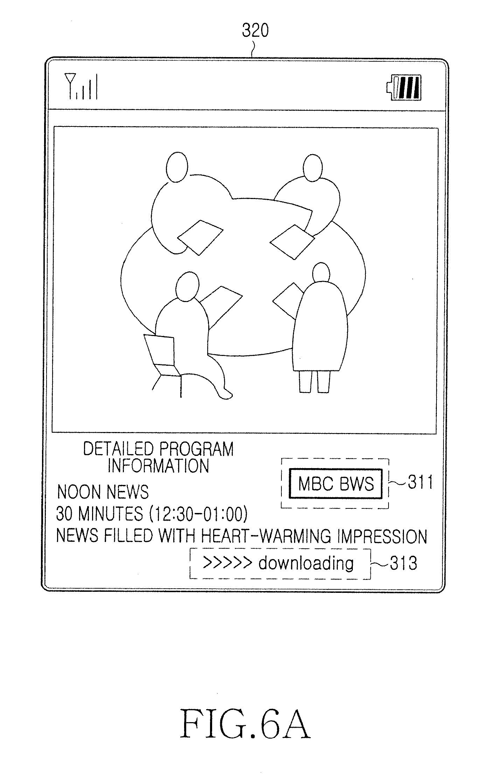

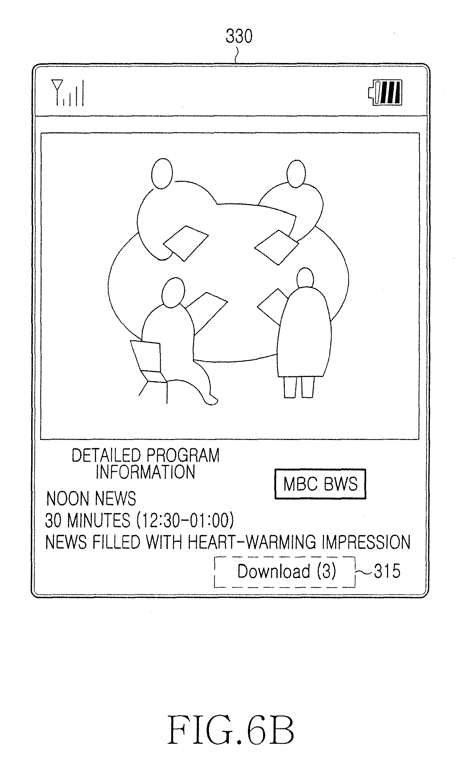

FIGS. 6A and 6B are views showing a configuration of an EPG sub-window when providing a digital multimedia broadcast according to the present invention. Referring to FIG. 6A, the EPG sub-window displayed on a display screen 320 displays detailed information on a current program, data service information 311 linked with the current program of a DMB channel and application service information 313. The application service information 313 displayed on the EPG sub-window of the display screen 320 indicates download status information, and the data service information 311 indicates linked Broadcasting Web Site (BWS) information. A display screen 330 of FIG. 6B displays included data service information 311 linked with an EPG sub-window and application service information 315. The application service information 315 displayed on the EPG sub-windows of the display screen 330 indicates the number of applications related to a corresponding channel. According to another embodiment of the present invention, the application service information 315 displayed on the EPG sub-windows of the display screen 330 may also include the name of an application, a download time point, automatic/selective download sorting information, automatic/selective execution sorting information and the like. For example, if a user requests more detailed application service information through a key operation in a state where only information on the number of download applications related to the current program of the correspondent channel is displayed on the EPG sub-window as shown in FIG. 6B, there may be provided the name of an application, a download time point, automatic/selective download sorting information, automatic/selective execution sorting information, download completion information and the like for each application. The download completion information indicates whether the correspondent application is downloaded and stored.

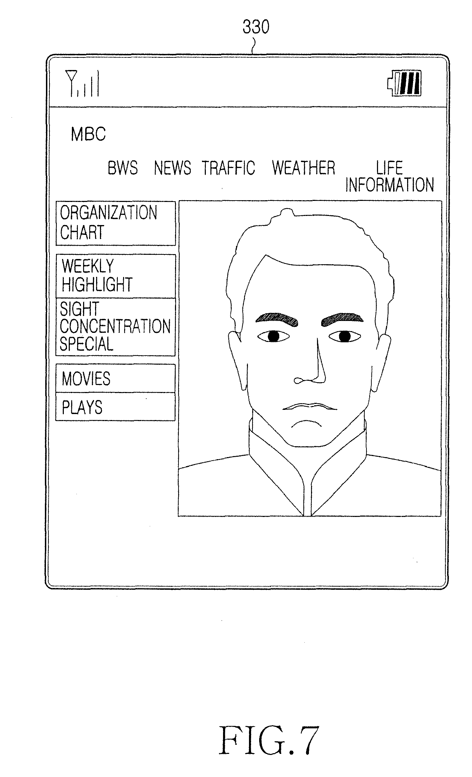

Referring back to FIG. 4A, in a state where the EPG sub-window is displayed, the DMB processor 20 identifies whether there is an input for requesting execution of a data service corresponding to data or application service information displayed on the EPG sub-window from the user at step 213. If any, the DMB processor 20 provides a corresponding data broadcasting service corresponding at the user's request at step 215. That is, the DMB processor 20 executes the corresponding service if the request for selectively executing the data or application service information provided together with the EPG data is input from the user. Referring to FIG. 6A, if there is a request for executing a linked BWS, the DMB processor 20 connects the linked BWS to provide a BWS screen 340, as shown in FIG. 7. Referring to FIG. 6B, a user may request detailed information on the application service and a download for each application contained in the detailed information through a key operation in a state where the information on the number of applications displayed on the EPG sub-window is selected through a key operation as shown on the display screen 330. The DMB processor 20 downloads an application corresponding to the request. Additionally, a user may request executing an application that has been previously downloaded and stored, and the DMB processor 20 may execute the application corresponding to the request.

Meanwhile, a user may request entire EPG information. Accordingly, the DMB processor 20 corresponds to the user's request at step 217 of FIG. 4B and displays data and application service information corresponding to EPG information for each channel at step 219.

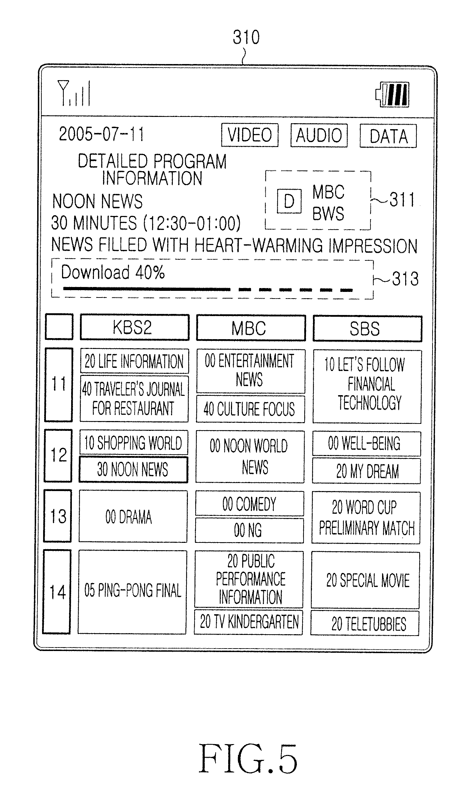

FIG. 5 is a view illustrating an entire EPG information display screen according to the present invention. The DMB processor 20 provides brief information on a program for each channel and time as shown on the display screen 310. If an arbitrary program information is selected among the information on the programs, the DMB processor 20 displays detailed information on the corresponding program, data service information 311 related thereto and download application information 313. Data and application service information provided together with the entire EPG information are also displayed in a format similar to the data and application service information displayed on the EPG sub-window, and data broadcasting service is executed corresponding to user's execution request. However, since data and application service related to DMB channels that are not connected currently are provided if the data and application service information are given together with the entire EPG information, the data service may not be executed depending on certain conditions. Accordingly, referring back to FIG. 4B, if the DMB processor 20 receives a data service execution request corresponding to the data or application information displayed together with the EPG information from the user at step 221, and proceeds to step 223. The DMB processor 20 identifies whether execution of the correspondent data service is possible at step 223. If possible, the DMB processor 20 proceeds to C and then to the step 215 of FIG. 4A so as to execute the corresponding data service. If not possible, the DMB processor 20 proceeds to step 225 so as to provide a message that execution of the corresponding data service is not possible.

As described above, the present invention provides brief information on a data broadcasting service together with EPG information and executes a corresponding data broadcasting service when there is a request for executing the corresponding data broadcasting service by selecting the brief information on the displayed data broadcasting service, so as to increase user convenience.

While the invention has been shown and described with reference to certain preferred embodiments thereof, it will be understood by those skilled in the art that various changes in form and details may be made therein without departing from the scope of the invention as defined by the appended claims.

## Claims

1. A method for providing brief information on a data broadcasting service in a Digital Multimedia Broadcasting, DMB, receiving terminal, the method comprising the steps of:
extracting brief information on a data broadcasting service from a received DMB signal to sort and store the brief information for each of at least one channel and program; and
displaying brief information on a relative data broadcasting service together with information for each program when requesting electronic rogram guide information.

2. The method as claimed in claim 1, wherein the brief information on the data broadcasting service includes data service information provided through an independent channel while linking with a main service of an arbitrary DMB channel and download application service information providing an arbitrary application module.

3. The method as claimed in claim 2, wherein the electronic program guide information request is one of an entire electronic program guide information display request and an electronic program guide sub-window display request for electronic program guide data related to a current program when connecting with an arbitrary channel.

4. The method as claimed in claim 3, further comprising selecting any one of the displayed data and application service information, and executing a corresponding data broadcasting service if there is a request for executing the data broadcasting service corresponding to the selected information.

5. The method as claimed in claim 4, wherein, if there is a request for executing a broadcast corresponding to the data service information, a data service provided through a linked channel is provided, and if there is a request for executing the application data broadcasting service, a download application service corresponding to the request for executing the application data broadcasting service is executed.

6. The method as claimed in claim 5, wherein the application data broadcasting service execution request is one part of a download request of an arbitrary application module and an execution request of an application that has been previously downloaded.

7. The method as claimed in claim 6, wherein the displayed application service information includes one or more of a number of applications to be downloaded, a name, a download time point, automatic/selective download sorting information, automatic/selective execution sorting information and download status information.

8. A device for providing brief information on a data broadcasting service in a DMB receiving terminal, the device comprising:
a memory for storing electronic program guide information and brief information on a data broadcasting service; and
a DMB processor for extracting the brief information on the data broadcasting service from received DMB signal to sort and store the brief information for each channel and program, and for displaying brief information on a relative data broadcasting service together with information for each program when requesting the electronic program guide information.

9. The device as claimed in claim 8, wherein the brief information on the data broadcasting service includes data service information serviced through an independent channel while linking with a main service of an arbitrary DMB channel and download application service information providing an arbitrary application module.

10. The device as claimed in claim 9, wherein the electronic program guide information request is one of an electronic program guide information display request and an electronic program guide sub-window display request for electronic program guide data related to a current program when connecting with an arbitrary channel.

11. The device as claimed in claim 10, wherein the DMB processor selects one of the displayed data and application service information, and executes a corresponding data broadcasting service if there is a request for executing the data broadcasting service corresponding to the selected information.

12. The device as claimed in claim 11, wherein, if there is a request for executing a broadcast corresponding to the data service information, the DMB processor provides a data service through a linked channel, and if there is a request for executing the application data broadcasting service, the DMB processor executes a download application service corresponding to the request for executing the application data broadcasting service.

13. The device as claimed in claim 12, wherein the application data broadcasting service execution request is one part of a download request of an arbitrary application module and an execution request of an application that has been previously downloaded.

14. The device as claimed in claim 13, wherein the displayed application service information includes one or more of a number of applications to be downloaded, a name, a download time point, automatic/selective download sorting information, automatic/selective execution sorting information and download status information.
